# EUROPÄISCHE PATENTSCHRIFT

(11) **EP 2 310 355 B1**
(45) Veröffentlichungstag und Bekanntmachung des Hinweises auf die Patenterteilung: **09.01.2013**
(21) Anmeldenummer: 09780487.6
(22) Anmeldetag: 13.07.2009
(51) Int. Cl.: C07C 211/36, C07C 209/48, C07C 209/52

(54) **5-ISOPROPYL-3-AMINOMETHYL-2-METHYL-1-AMINO-CYCLOHEXAN (CARVONDIAMIN) UND EIN VERFAHREN ZU DESSEN HERSTELLUNG**
5-ISOPROPYL-3-AMINOMETHYL-2-METHYL-1-AMINO-CYCLOHEXANE (CARVONE DIAMINE), AND METHOD FOR THE PRODUCTION THEREOF
5-ISOPROPYL-3-AMINOMÉTHYL-2-MÉTHYL-1-AMINO-CYCLOHEXANE (CARVONE DIAMINE) ET PROCÉDÉ DE PRODUCTION DE CELUI-CI

(30) Priorität: 25.07.2008 EP 08161181
(43) Veröffentlichungstag der Anmeldung: 20.04.2011
(73) Patentinhaber: BASF SE, 67056 Ludwigshafen (DE)
(72) Erfinder: ERNST, Martin, 69115 Heidelberg (DE); OFTRING, Alfred, 67098 Bad Dürkheim (DE); WISSEL-STOLL, Kathrin, 67056 Ludwigshafen (DE); ÜNSAL, Ömer, 55128 Mainz (DE); FREYER, Stephan, 67434 Neustadt (DE); ALTENHOFF, Ansgar Gereon, 69117 Heidelberg (DE); STAFFEL, Wolfgang, 67165 Waldsee (DE)
(86) Internationale Anmeldenummer: PCT/EP2009/058888
(87) Internationale Veröffentlichungsnummer: WO 2010/009995

(56) Entgegenhaltungen:
- EP-A- 0 128 382
- EP-A- 0 895 984
- EP-A- 0 976 723
- WO-A-2004/060866
- DE-A1- 19 836 474

## Beschreibung

Die vorliegende Erfindung betrifft 5-Isopropyl-3-aminomethyl-2-methyl-1-amino-cyclohexan (Carvondiamin) sowie ein Verfahren zu dessen Herstellung durch a) Umsetzung von Carvon mit Cyanwasserstoff, b) anschließender Umsetzung des in Stufe a) erhaltenen Carvonnitrils mit Ammoniak in Gegenwart eines Iminbildungskatalysators und c) nachfolgender Umsetzung des in Stufe b) erhaltenen Carvonnitrilimin-haltigen Reaktionsgemisches mit Wasserstoff und Ammoniak an Hydrierkatalysatoren. Die vorliegende Erfindung betrifft weiterhin die Verwendung von Carvondiamin.

Cycloaliphatische Diamine finden als Härter bei Epoxyharzen, als Zwischenprodukt bei der Herstellung von Diisocyanaten - die bei der Herstellung von Polyurethanen eine wichtige Rolle spielen -, als Starter bei der Herstellung von Polyetherolen sowie als Monomere für die Polyamidherstellung Verwendung.

Die Struktur des eingesetzten Diamins kann die Eigenschaften der aus den Diaminen hergestellten Polymermaterialien, wie Wetterbeständigkeit, Hydrolysebeständigkeit, Chemikalienbeständigkeit, Lichtbeständigkeit, elektrische sowie mechanische Eigenschaften beeinflussen. Sie kann aber auch einen Einfluss auf die Verarbeitbarkeit und die Verarbeitung der Diamine zu den entsprechenden Polymermaterialien- beispielsweise der Aushärtung von Epoxyharzen - ausüben.

Industriell eingesetzte cycloaliphatische Diamine sind beispielsweise Isophorondiamin (IPDA), Bis(4-aminocyclohexyl)methan (PACM) und 1,2-Diaminocyclohexan (DACH). PACM und DACH können durch Hydrierung der entsprechenden aromatischen Verbindungen, wie 4,4'-Diaminodiphenylmethan bzw. o-Phenylendiamin, synthetisiert werden. DACH fällt weiterhin als Nebenprodukt in der Hexamethylendiamin-Produktion an.

Bei DACH und PACM sind beide Aminogruppen direkt am aliphatischen Ring substituiert und weisen somit die gleiche, oder zumindest eine ähnliche Reaktivität auf. Für gewisse Anwendungen und Eigenschaften kann es vorteilhaft sein, wenn die beiden Aminogruppen des Diamins eine unterschiedliche Reaktivität aufweisen, da hierdurch das Verarbeitungs- und Aushärtungsverhalten beeinflusst wird. Beispielsweise sitzt bei IPDA eine Aminogruppe direkt am aliphatischen Ring, während die andere Aminogruppe über eine Methylenbrücke mit dem aliphatischen Ring verknüpft ist. IPDA wird in der Regel durch Addition von Cyanwassterstoff an Isophoron zu Isophoronnitril und anschließender Hydrierung in Gegenwart von Ammoniak zu IPDA hergestellt.

In EP-A1-0394058 werden ebenfalls cycloaliphatische Diamine mit unterschiedlich reaktiven Aminogruppen offenbart. Durch Nitrierung von Alkylphenylketonen werden Nitrophenylalkylketone erhalten, welche in Gegenwart von Wasserstoff und Ammoniak zu (Aminophenyl)alkylaminen umgesetzt werden. Diese werden anschließend zu den entsprechenden (Aminocyclohexyl)alkylaminen reduziert. Die Ausbeute in der letzten Hydrierstufe liegt ungefähr bei 80 bis 90%.

In EP-A1-0895984 wird die Herstellung von cycloaliphatischen Diaminen mit Aminogruppen unterschiedlicher Reaktivität durch reduktive Aminierung von alkylsubstituierten 3-Formylcycloalkanonen beschrieben. Alkylsubstituierte 3-Formylcycloalkanone sind wiederum durch Umsetzung von alkylsubstituierten Cycloalkenonen und Formaldehyd erhältlich. Ausgehend von den eingesetzten Cycloalkenonen beträgt die Ausbeute an alkylsubstituierten, cycloaliphatischen Diaminen im Bereich von 50 bis 60%.

Die DE-A-19836474 und die WO 2004/060866 betreffen die Herstellung von Isophorondiamin durch Hydrocyanierung von Isophoron und die anschließende aminierende Hydrierung.

In der EP-A-0976723 und der EP-A-0128382 werden 3-Aminomethylcycloalkylamine und deren Anwendungen offenbart.

IPDA wird üblicherweise aus Aceton hergestellt, während die zuvor genannten alkylsubstituierten Cycloalkyldiamine in der Regel auf Cyclohexen basieren. Sowohl Aceton als auch Cyclohexen werden im Allgemeinen aus dem petrochemischen Rohstoff Naphtha erhalten.

Aufgabe der vorliegenden Erfindung war die Bereitstellung eines cycloaliphatischen Diamins, das auch auf Basis von nachwachsenden Rohstoffen hergestellt werden kann. Das Zurückgreifen auf nachwachsende Rohstoffe kann zu einer Schonung von erschöpfbaren Ressourcen beitragen und ermöglicht ein nachhaltiges Wirtschaften. Ein weitere Aufgabe der vorliegende Erfindung bestand darin, ein cycloaliphatisches Diamin zu synthetisieren, das ähnlich wie IPDA, sowohl eine Aminogruppe aufweist, die direkt am aliphatischen Ring gebunden ist, und eine Aminogruppe aufweist, die über eine Methylengruppe mit dem aliphatischen Ring verknüpft ist. Somit sollte ein Diamin auf Basis von nachwachsenden Rohstoffen erhalten werden, dass eine ähnliche Reaktivität wie IPDA aufweist und in vielen Fällen als Substitut für IPDA eingesetzt werden kann.

Eine weitere Aufgabe bestand darin, ein neues Diamin zur Verfügung zu stellen, um das Eigenschaftsprofi von Anwendungen, in denen Diamine eingesetzt werden, zu steuern und zu kontrollieren. In vielen Anwendungen, beispielsweise dem Einsatz des Diamins als Härter bei Epoxyharzen, als Zwischenprodukt bei der Herstellung von Polyurethanen, als Starter bei der Herstellung von Polyetherolen sowie als Monomer für die Polyamidherstellung, werden aufgrund der vielfältigen Anforderungen und der unterschiedlichen Einsatzgebiete eine Variation an verschiedenen Grundbausteinen benötigt, um gezielt Eigenschaften, wie Wetterbeständigkeit, Hydrolysebeständigkeit, Chemikalienbeständigkeit, Lichtbeständigkeit, elektrische sowie mechanische Eigenschaften, einstellen und beeinflussen zu können.

Zudem sollte ein Verfahren zur Herstellung von Carvondiamin zur Verfügung gestellt werden, bei dem nur wenig Nebenprodukte gebildet werden, die schwer aus dem Reaktionsgemisch abzutrennen sind, beispielsweise die Bildung von Dimeren, die bei der reduktiven Aminierung durch Kopplung von zwei Nitril- oder Ketogruppen unterschiedlicher Moleküle entstehen können. Insbesondere sollte ein hoher Nitrilumsatz bzw. Sättigungsgrad des Reaktionsproduktes erzielt werden, da Nitrilamine oder Aminoimine die Eigenschaften der polymeren Materialien verschlechtern und C-C-Doppelbindungen die Farbechtheit beeinträchtigen können. Teilgesättigte Verbindungen sind in der Regel zudem nur schwer vom gesättigten Reaktionsprodukt abzutrennen.

Weiterhin sollte eine hohe Verfahrenökonomie durch Erzielung hoher Ausbeuten und Selektivitäten erreicht werden.

Demgemäß wurde die Verbindung 5-Isopropyl-3-aminomethyl-2-methyl-1-amino-cyclohexan (Carvondiamin) der Formel (I) 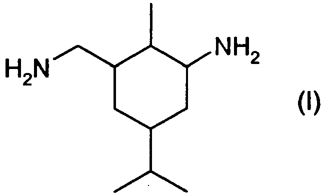 gefunden.
Ein weiterer Gegenstand der vorliegenden Erfindung ist ein Verfahren zur Herstellung von Carvondiamin der Formel (I) durch
a) Umsetzung von Carvon der Formel (II) 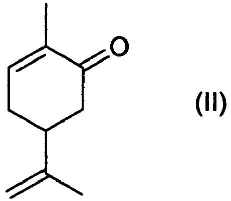 mit Cyanwasserstoff in Gegenwart eines basischen Katalysators zu Carvonnitril der Formel (III), 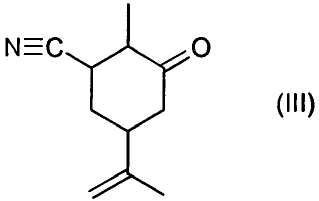
b) Umsetzung des in Stufe a) erhaltenen Carvonnitrils mit Ammoniak in Gegenwart eines Iminbildungskatalysators zu Carvonnitrilimin der Formel (IV), und 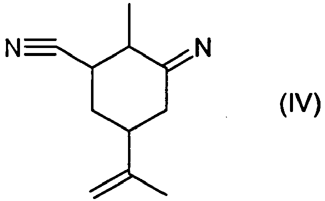
c) Umsetzung des in Stufe b) erhaltenen Carvonnitrilimin-haltigen Reaktionsgemisches mit Wasserstoff und Ammoniak an Hydrierkatalysatoren.

In das erfindungsgemäße Verfahren zur Herstellung von Carvondiamin wird Carvon als Ausgangsstoff eingesetzt. Die Herstellung bzw. Gewinnung von Carvon ist beispielsweise in Ullmann's Encyclopedia of Industrial Chemistry, Verlag Chemie, Electronic Edition Release 2008, 7th Edition, Kapitel "Flavor and Fragrances" beschrieben. So kann Carvon beispielsweise durch fraktionierende Destillation von Kümmel- oder Pfefferminzöl gewonnen oder durch Oxidation von Limonen erhalten werden. Limonen wiederum kann durch Naturstoffextraktion erhalten werden. So fällt Limonen beispielsweise in großen Mengen als Nebenprodukt bei der Orangensaftproduktion oder bei der säurekatalysierten Isomerisierung von α- und β- Pinen an.

Carvon kann in Form der reinen Enantiomere (R-(-)- Carvon oder S-(+)-Carvon), als racemisches oder als entantiomer-angereichertes Gemisch in das Verfahren eingesetzt werden.

R-(-)- Carvon wird in der Regel aus Limonen aus Orangenschalen durch Oxidation mit NOCl und Eliminierung von HCl hergestellt (R.H. Reitsema, Journal of Organic Chemistry, 1958, 2039). R-(-)-Carvon ist auch aus Krauseminze (Spearmint) erhältlich. Das Enantiomere S-(+)-Carvon kommt im Kümmelöl und Dillöl vor. Racemisches Carvon kann wie R-(-)-Carvon aus racemischem Limonen, das aus Säure-katalysierter Isomerisierung von Pinen oder Diels-Alder-Reaktion von Isopren gewonnen werden kann, hergestellt werden. Weitere Methoden sind die allylische Oxidation mit Pd (II)-Salzen in Gegenwart von Cu(II) und Luftsauerstoff (WO-A-99/12880) oder die unkatalysierte Luftoxidation von Limonen. Üblicherweise wird Carvon in einer Reinheit von mehr als 90%, bevorzugt mehr als 95% und besonders bevorzugt mehr als 98% eingesetzt.

Als weiterer Einsatzstoff wird Cyanwasserstoff (HCN) in das erfindungsgemäße Verfahren eingesetzt. Für die Herstellung von HCN sind im wesentlichen die folgenden Verfahren von Bedeutung: bei der Ammonoxidation von Methan (Andrussow-Verfahren) wird ein Gemisch aus Ammoniak und Methan bei rund 1200°C an einem Platinnetz als Katalysator oxidiert; bei der Ammondehydrierung von Methan (Degussa-BMA-Verfahren) werden Ammoniak und Methan mit Hilfe eines Katalysators zu Blausäure und Wasserstoff umgesetzt und bei der Formamid-Spaltung (BASF-Verfahren) wird Formamid verdampft und stark erhitzt, wobei sich Formamid in Blausäure und Wasser spaltet.

Die Hydrocyanierung von Carvon ist z.B. in Chemistry & Industry (London, UK), 1967, (2), 1175 beschrieben.

In dem erfindungsgemäßen Verfahren zur Herstellung von Carvondiamin wird in einer ersten Stufe a) Carvon (5-Isopropenyl-2-methyl-cyclohex-2-enon) mit Cyanwasserstoff (HCN) in Gegenwart eines basischen Katalysators umgesetzt.

Die Umsetzung von Carvon mit Cyanwasserstoff findet in Gegenwart eines basischen Katalysators statt. Als basische Katalysatoren sind alle Substanzen geeignet, die unter den Reaktionsbedingungen in Gegenwart von Cyanwasserstoff Cyanidionen bilden oder enthalten. Dazu gehören z. B. Hydroxide, Cyanide und Alkoholate der Alkali- und Erdalkalimetalle sowie quarternäre Ammoniumverbindungen. Vorzugsweise werden Alkalicyanide, Alkalihydroxide, Erdalkalihydroxide und C₁- bis C₄-Alkalialkoholate wie Natriummethylat, Natriumethylat, Kaliummethylat, Kalium-tert.-Butylat, Lithiummethylat, besonders bevorzugt wird Natriummethylat verwendet. Ganz besonders bevorzugt wird NaCN verwendet, das z.B. ohne Isolierung durch Inkontaktbringen von NaOH und HCN hergestellt werden kann.

Die Katalysatorkonzentration liegt zwischen 0,01 und 3 Gew.-% bezogen auf das Reaktionsgemisch. Vorzugsweise wird die Katalysatorkonzentration so gewählt, dass die von der Reaktionstemperatur und der Zusammensetzung des Reaktionsgemisches abhängige Löslichkeit des basischen Katalysators nicht überschritten wird, das sind vorzugsweise Konzentrationen zwischen 0,01 und 0,3 Gew.-% bezogen auf das Reaktionsgemisch.

Die Umsetzung von Carvon mit Cyanwasserstoff kann bei Reaktionstemperaturen von 80 bis 220°C, bevorzugt 100 bis 180°C, besonders bevorzugt 120 bis 170°C, durchgeführt werden.

Der Reaktionsdruck (absolut gemessen) beträgt in der Regel 0,05 bis 2 MPa, bevorzugt 0,09 bis 1 MPa, besonders bevorzugt Atmosphärendruck (Normaldruck) bis 3 bar. Der Druck kann z.B. durch Aufpressen von Inertgasen (Stickstoff) erzeugt werden.

Bei der erfindungsgemäßen Umsetzung von Carvon mit Cyanwasserstoff wird in der Regel Carvon im molaren Überschuss bezogen auf den Cyanwasserstoff eingesetzt. Das molare Verhältnis Carvon:HCN der beiden Einsatzstoffe Carvon und Cyanwasserstoff (HCN) beträgt üblicherweise 2:1 bis 10:1, bevorzugt 2:1 bis 5:1, besonders bevorzugt 2:1 bis 3:1.

Die Umsetzung kann in Gegenwart oder Abwesenheit von inerten Lösungsmitteln vorgenommen werden.

Als inerte Lösungsmittel für die Umsetzung eignen sich Wasser und C₁- bis C₂₀-Alkanole, bevorzugt C₁- bis C₈-Alkanole, besonders bevorzugt C₁- bis C₄-Alkanole, wie Methanol, Ethanol, n-Propanol, iso-Propanol, n-Butanol, iso-Butanol und tert.-Butanol, aliphatische Kohlenwasserstoffe mit 5 bis 30 C-Atomen, bevorzugt mit 5 bis 20 C-Atomen, besonders bevorzugt mit 5 bis 10 C-Atomen, wie n-Pentan, Pentanisomerengemische, n-Hexan, Hexanisomerengemische, n-Heptan, Heptanisomerengemische, n-Octan, Octanisomerengemische, cycloaliphatische Kohlenwasserstoffe mit 5 bis 20 C-Atomen, bevorzugt mit 5 bis 12 C-Atomen, besonders bevorzugt mit 5 bis 8 C-Atomen, wie Cyclopentan, Cyclohexan, Cycloheptan und Cyclooctan, Amide, wie Dimethylformamid, Dimethylacetamid, N-Methylpyrrolidon, Harnstoffe, wie N,N'-Dimethylethylenharnstoff, N,N'-Dimethylpropylenharnstoff, N,N,N',N'-Tetra-n-butylharnstoff, oder Carbonate, wie Ethylencarbonat und Propylencarbonat.

Besonders bevorzugt verwendet man Carvon im molaren Überschuss, bezogen auf HCN, und setzt kein externes Lösungsmittel zu.

Die erfindungsgemäße Umsetzung von Carvon mit Cyanwasserstoff kann diskontinuierlich, semikontinuierlich oder bevorzugt kontinuierlich durchgeführt werden.

Als Reaktionsgefäße bzw. Reaktoren eignen sich beispielsweise Rührreaktoren, Rohrreaktoren, Rührbehälterkaskaden, Schlaufenreaktoren oder Mischkreise.

Beispielsweise kann eine kontinuierliche Verfahrensdurchführung derart erfolgen, dass der basische Katalysator, gegebenenfalls in einem inerten Lösungsmittel oder in Carvon gelöst, kontinuierlich in eine Apparatur gespeist wird, in der kontinuierlich Carvon mit Cyanwasserstoff unter Normaldruck oder unter erhöhtem Druck (0,09 bis 1 MPa, absolut gemessen) umgesetzt wird.

Üblicherweise wird die Umsetzung in einer mehrstufigen Rührkesselkaskade durchgeführt.

Es ist jedoch auch möglich die Umsetzung in zwei getrennten Reaktionszonen durchzuführen, wobei die erste Reaktionszone im Wesentlichen vollständige Rückvermischung aufweist und die zweite Reaktionszone im Wesentlichen keine Rückvermischung aufweist.

Als Reaktor für die erste Reaktionszone mit im Wesentlichen vollständiger Rückvermischung kann z. B. ein Rührkessel, ein Mischkreis oder ein Schlaufenreaktor eingesetzt werden. Die frei werdende Reaktionswärme wird über geeignete Wärmetauscher abgeführt.

Als Reaktor für die zweite Reaktionszone, der im Wesentlichen keine Rückvermischung aufweist, eignen sich zylindrische Reaktoren mit Füllkörpern oder fest angeordneten Einbauten, die eine Rückvermischung vollständig oder teilweise verhindern. Bei der Durchführung der Synthese im Labormaßstab kann jedoch auch ein Rohrreaktor eingesetzt werden, der im turbulenten Strömungsbereich betrieben wird.

Die für vollständigen HCN-Umsatz notwendige Verweilzeit ist abhängig von der Reaktionstemperatur und der Katalysatorkonzentration. Sie beträgt für den Rührreaktor in aller Regel 1 bis 4 Stunden, für den ohne Rückvermischung betriebenen Nachreaktor in aller Regel 0,2 bis 1,5 Stunden.

Eine diskontinuierliche bzw. semikontinuierliche Verfahrensführung kann derart erfolgen, dass
a) Carvon mit dem basischen Katalysator vorgelegt und Cyanwasserstoff in einem inerten Lösungsmittel oder in Carvon zugeben wird, oder
b) Carvon mit Cyanwasserstoff vorgelegt und der basische Katalysator in einem inerten Lösungsmittel oder Carvon zugeben wird, oder
c) Carvon vorgelegt und Cyanwasserstoff und der basische Katalysator in einem inerten Lösungsmittel oder Carvon zugeben wird.

Die Variante a) ist hierbei bevorzugt.

Das durch Umsetzung von Carvon mit Cyanwasserstoff erhaltene Reaktionsgemisch enthält Carvonnitril (5-Isopropenyl-3-cyano-2-methyl-cyclohexanon) der Formel (III).

Das erhaltene Reaktionsgemisch kann mit Wasser extrahiert werden, um den gelösten Katalysator zu entfernen.

Der basische Katalysator kann jedoch auch durch Zusatz einer äquivalenten oder überschüssigen Menge einer organischen oder anorganischen Säure neutralisiert werden.

Zur Neutralisation des Reaktionsaustrages können Säuren, z.B. anorganische Säuren, wie Phosphorsäure und Schwefelsäure, oder organische Säuren, beispielsweise Sulfonsäuren, wie Methansulfonsäure, Toluolsulfonsäure, Carbonsäuren, wie Ameisensäure, Essigsäure, Propionsäure, Malonsäure, 2-Ethyl-hexansäure und Adipinsäure, verwendet werden.

Das Reaktionsgemisch kann anschließend, ggf. nach erfolgter Extraktion mit Wasser oder Neutralisation, durch fraktionierte Destillation gereinigt werden.

Man erhält Carvonnitril (5-Isopropenyl-3-cyano-2-methyl-cyclohexanon) der Formel (III).

Nicht umgesetztes Carvon kann in den Reaktionsprozess zurückgeführt werden.

In Stufe b) wird das aus der Stufe a) erhaltene Carvonnitril mit überschüssigem Ammoniak in Gegenwart eines Iminbildungskatalysators umgesetzt (Iminierung).

Als Iminbildungskatalysator kommen beispielsweise feste Brönstedt- oder LewisSäuren in Betracht, wie sie beispielsweise in der EP-A1-449089 (Seite 2, Spalte 2, Zeilen 11-20) und in dem Artikel von Tanabe et al. (K. Tanabe, Studies in Surface Science and Catalysis, Vol. 51, 1989,. S. 1 ff) beschrieben sind. Beispielhaft seien hier acide Metalloxidkatalysatoren, wie Aluminiumoxid, Titandioxid, Zirkoniumdioxid und Siliciumdioxid genannt. Weiterhin kommen mit Ammonium-lonen beladene anorganische oder organische Ionenaustauscher, wie Zeolithe oder sulfonierte Copolymerisate aus Styrol und Divenylbenzol (z.B. der Marken Lewatit® der Fa. Lanxess, Amberlite® der Fa. Rohm & Haas) oder Ionenaustauscher auf Basis Siloxan (z.B. der Marke Deloxan® der Fa. Degussa) in Betracht.

Pro Mol eingesetztes Carvonnitril werden üblicherweise 5 bis 500 Mol Ammoniak (NH₃), bevorzugt 10 bis 400 Mol NH₃, besonders bevorzugt 20 bis 300 Mol NH₃, eingesetzt.

Die Iminierung von Carvonnitril kann in Anwesenheit eines Lösungsmittels erfolgen, z.B. in Alkanolen oder Ethern, wie Ethanol, Butanol oder Tetrahydrofuran (THF). Bevorzugt wird die Iminierung von Carvonnitril ohne eines Zusatzes von Lösungsmittel durchgeführt.

Die Iminierung kann diskontinuierlich oder bevorzugt kontinuierlich durchführt werden. Die diskontinuierliche Iminierung kann beispielsweise in einem Rührautoklaven, einer Blasensäule oder einem Umlaufreaktor, wie etwa einem Strahlschlaufenreaktor, durchgeführt werden.

Bei der diskontinuierlichen Iminierung wird üblicherweise eine Suspension von Carvonnitril und Katalysator im Reaktor vorgelegt. Um einen hohen Umsatz und hohe Selektivität zu gewährleisten, wird die Suspension von Carvonnitril und Katalysator mit Ammoniak üblicherweise gut gemischt werden, z.B. durch einen Turbinenrührer in einem Autoklaven. Das suspendierte Katalysatormaterial kann mit Hilfe gebräuchlicher Techniken eingebracht und wieder abgetrennt werden (Sedimentation, Zentrifugation, Kuchenfiltration, Querstromfiltration). Der Katalysator kann einmal oder mehrmals verwendet werden.

Die Katalysatorkonzentration beträgt vorteilhaft 0,1 bis 50 Gew.-%, bevorzugt 0,5 bis 40 Gew.-%, besonders bevorzugt 1 bis 30 Gew.-%, insbesondere 5 bis 20 Gew.-%, jeweils bezogen auf das Gesamtgewicht der Suspension bestehend aus Carvonnitril und Katalysator.

Die mittlere Katalysatorpartikelgröße beträgt vorteilhaft im Bereich von 0,001 bis 1 mm, bevorzugt im Bereich von 0,005 bis 0,5 mm, insbesondere 0,01 bis 0,25 mm.

Die Iminierung wird bevorzugt kontinuierlich durchgeführt, üblicherweise in Druckbehältern oder Druckbehälterkaskaden. Bevorzugt werden Carvonnitril und NH₃ durch einen Rohrreaktor geleitet, in dem der Iminbildungskatalysator in Form eines Festbetts angeordnet ist.

In der Regel stellt man bei der kontinuierlichen Iminierung eine Katalysatorbelastung von 0,01 bis 10, vorzugsweise von 0,05 bis 7, besonders bevorzugt von 0,1 bis 5 kg Carvonnitril pro kg Katalysator und Stunde ein.

Die Iminierung wird bevorzugt in einem Temperaturbereich von 20 bis 150°C, vorzugsweise 30 bis 130°C und besonders bevorzugt bei 50 bis 100°C durchgeführt. Der Druck bei der Iminierung beträgt in der Regel von 50 bis 300 bar, bevorzugt 100 bis 250 bar.

Das Reaktionsgemisch aus der Iminierung enthält üblicherweise Carvonnitrilimin und Ammoniak und nicht umgesetztes Carvonnitril. Der Umsatz von Carvonnitril zu Carvonnitrilimin liegt üblicherweise bei mehr als 80%, vorzugsweise mehr als 90% und besonders bevorzugt mehr als 95%.

Das Reaktionsgemisch aus Stufe b) wird in Stufe c) mit Wasserstoff und Ammoniak an Hydrierkatalysatoren umgesetzt (reduktive Aminierung).

Die Umsetzung des Reaktionsgemisches enthaltend Carvonnitrilimin erfolgt bevorzugt in flüssigem Ammoniak. Pro Mol Carvonnitrilimin setzt man üblicherweise 5 bis 500 Mol NH₃, bevorzugt 10 bis 400 Mol NH₃ und besonders bevorzugt 20 bis 300 Mol NH₃ ein. Zweckmäßigerweise stellt man bei der vorgelagerten Iminierung das Molverhältnis zwischen Carvonnitril und NH₃ so ein, dass das Molverhältnis auch bei der reduktiven Aminierung in einem geeigneten Bereich liegt. Der NH₃-Anteil kann jedoch vor der reduktiven Aminierung durch Zugabe von zusätzlichem NH₃ auf einen gewünschten Wert erhöht werden.

Als weiterer Ausgangsstoff für die Umsetzung des Carvonnitrilimin-haltigen Reaktionsgemisches wird Wasserstoff eingesetzt. Das Molverhältnis zwischen Wasserstoff und Carvonnitrilimin beträgt in der Regel 3 bis 10 000 zu 1, bevorzugt von 4 bis 5000 zu 1 und besonders bevorzugt von 5 bis 1000 zu 1.

Der Wasserstoff wird dem Carvonnitrilimin-haltigen Reaktionsgemisch bevorzugt nach der Iminierung und vor der reduktiven Aminierung zugeführt. Es ist jedoch auch denkbar, dass der Wasserstoff bereits vor der Iminierung zugeführt wird, da die Iminierung üblicherweise an Katalysatoren erfolgt, die die Hydrierung nicht katalysieren. Somit kann auch vor der Iminierung zugeführter Wasserstoff als Ausgangsstoff für die Umsetzung des Carvonnitrilimin-haltigen Reaktionsgemisches während der reduktiven Aminierung zur Verfügung stehen.

Als Hydrierkatalysatoren können prinzipiell alle Hydrierkatalysatoren eingesetzt werden, die Nickel, Kobalt, Eisen, Kupfer, Ruthenium, Palladium, Platin, Rhodium und/oder andere Metalle der VIII. Nebengruppe des Periodensystems enthalten. Als Hydrierkatalysatoren sind weiterhin Katalysatoren geeignet, die die Elemente Chrom, Mangan, Molybdän, Wolfram und/oder Rhenium enthalten.

Bevorzugt verwendet man Hydrierkatalysatoren, die Ruthenium, Kobalt und/oder Nickel enthalten. Besonders bevorzugt sind Katalysatoren die Ruthenium und/oder Kobalt enthalten.

Die oben genannten Hydrierkatalysatoren können in üblicher Weise mit Promotoren, beispielsweise mit Chrom, Eisen, Kobalt, Mangan, Thallium, Molybdän, Titan und/oder Phosphor dotiert werden.

Die katalytisch aktiven Metalle können als Vollkontakte oder auf Trägern eingesetzt werden. Als solche Träger kommen z.B. Alumumiumoxid, Titandioxid, Zirkoniumdioxid oder Magnesiumoxid/Aluminiumoxid in Betracht. Die Träger können auch iminieraktiv sein, um die Umsetzung von während der Hydrierung der Imingruppe im Gleichgewicht mit dem Imin vorliegendem Keton zu ermöglichen.

Die in das erfindungsgemäße Verfahren einsetzbaren Hydrierkatalysatoren werden in der Regel durch Reduktion von so genannten Katalysatorvorläufem mit Wasserstoff erhalten.

Der Katalysatorvorläufer enthält üblicherweise sauerstoffhaltige Verbindungen der oben genannten Metalle.

Die Katalysatorvorläufer können nach bekannten Verfahren, z.B. durch Fällung, Auffällung oder Imprägnierung hergestellt werden.

Solche Katalysatorvorläufer sind beispielsweise in EP-A-0636409 offenbarte Katalysatoren, deren katalytisch aktive Masse vor der Reduktion mit Wasserstoff 55 bis 98 Gew.-% Co, berechnet als CoO, 0,2 bis 15 Gew.-% Phosphor, berechnet als H₃PO₄, 0,2 bis 15 Gew.-% Mangan, berechnet als MnO₂, und 0,2 bis 15 Gew.-% Alkali, berechnet als M₂O (M=Alkalimetall), enthält, oder

in EP-A-0742045 offenbarte Katalysatoren, deren katalytisch aktive Masse vor der Reduktion mit Wasserstoff 55 bis 98 Gew.-% Co, berechnet als CoO, 0,2 bis 15 Gew.-% Phosphor, berechnet als H₃PO₄, 0,2 bis 15 Gew.-% Mangan, berechnet als MnO₂, und 0,05 bis 5 Gew.-% Alkali, berechnet als M₂O (M=Alkalimetall), enthält, oder

in EP-A-696572 offenbarte Katalysatoren, deren katalytisch aktive Masse vor der Reduktion mit Wasserstoff 20 bis 85 Gew.-% ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃, und 0 bis 10 Gew.-% sauerstoffhaltige Verbindungen des Aluminiums und/oder Mangans, berechnet als Al₂O₃ bzw. MnO₂, enthält, beispielsweise der in loc. cit, Seite 8, offenbarte Katalysator mit der Zusammensetzung 31,5 Gew.-% ZrO₂, 50 Gew.-% NiO, 17 Gew.-% CuO und 1,5 Gew.-% MoO₃.

Die katalytisch aktiven Metalle können auch in Form von Schwammkatalysatoren, sogenannten Raney-Katalysatoren, eingesetzt werden. Als Raney-Katalysatoren werden bevorzugt Raney-Kobalt-Katalysatoren, Raney-Nickel-Katalysatoren und/oder Raney-Kupfer-Katalysatoren eingesetzt. Besonders bevorzugt werden Raney-Kobalt-Katalysatoren verwendet.

Als Hydrierkatalysatoren können auch vorteilhaft selektive Hydrierkatalysatoren eingesetzt werden, wobei unter selektiven Hydrierkatalysatoren solche Katalysatoren zu verstehen sind, die bevorzugt die Imingruppe gegenüber der Nitrilgruppe des Carvonnitrilimins hydrieren.

Selektive Hydrierkatalysatoren sind beispielsweise Hydrierkatalysatoren, die Ruthenium, Palladium und/oder Rhodium enthalten. Bevorzugte selektive Hydrierkatalysatoren enthalten Ruthenium und/oder Rhodium und besonders bevorzugte selektive Hydrierkatalysatoren enthalten Ruthenium.

Die reduktive Aminierung wird vorzugsweise in Gegenwart einer basischen Verbindung und/oder einem basischen Hydrierkatalysator durchgeführt.

Dabei versteht sich, dass der Begriff basische Verbindung nicht das Edukt Ammoniak erfasst, sondern eine oder mehrere der untenstehend aufgeführten Verbindungen umfasst oder solche Verbindungen, die in analoger Weise wie die untenstehend aufgeführten Verbindungen wirken.

Als geeignete basische Verbindungen kommen basische Metallverbindungen, wie die Oxide, Hydroxide oder Carbonate der Alkali-, Erdalkalkali- oder Seltenerdmetalle in Betracht.

Bevorzugt sind die Metallverbindungen der Alkali- und Erdalkalimetalle, wie die entsprechenden Oxide, Hydroxide und Carbonate, wie Li₂O, Na₂O, K₂O, Rb₂O, Cs₂O, Li-OH, NaOH, KOH, RbOH, CsOH, Li₂CO₃, Na₂CO₃, K₂CO₃, Cs₂CO₃, Rb₂CO₃, MgO, CaO, SrO, BaO, Mg(OH)₂, Ca(OH)₂, Sr(OH)₂, Ba(OH)₂, MgCO₃, CaCO₃, SrCO₃ oder BaCO₃. Besonders bevorzugt sind LiOH, NaOH oder KOH.

Ebenfalls geeignete, bevorzugte basische Verbindungen sind Amine oder Ammoniumhydroxide.

Besonders bevorzugt werden Lösungen der basischen Verbindungen in Wasser oder anderen geeigneten Lösungsmitteln, wie Alkanolen, wie C₁-C₄-Alkanolen, z.B. Methanol oder Ethanol, oder Ether, wie cyclische Ether, z.B. THF oder Dioxan, dem Reaktionsgemisch zugefügt. Besonders bevorzugt werden Lösungen von Alkali- oder Erdalkalihydroxiden in Wasser, besonders bevorzugt Lösungen von LiOH, NaOH oder KOH in Wasser, zugegeben.

Bevorzugt beträgt die Konzentration der basischen Verbindung in Wasser oder anderen geeigneten Lösungsmitteln 0,01 bis 20 Gew.-%, bevorzugt 0,1 bis 10 und besonders bevorzugt 0,2 bis 5 Gew.-%.

Die Menge der zugefügten Lösung der basischen Verbindung wird üblicherweise so gewählt, dass das Verhältnis der Masse der zugefügten basischen Verbindung zur Masse des Carvonnitrilimins im Reaktionsgemisch 100 bis 10 000 zu 1 000 000 beträgt, bevorzugt 150 bis 5000 zu 1 000 000 und besonders bevorzugt 200 bis 1000 zu 1 000 000.

Die reduktive Aminierung kann auch in Gegenwart von basischen Hydrierkatalysatoren erfolgen. Solche basischen Hydrierkatalysatoren sind obengenannte Hydrierkatalysatoren, welche mit basischen Komponenten, wie Oxiden oder Hydroxiden von Alkali-, Erdalkali- und Seltenerdmetallen, dotiert und/oder auf basischen Trägern aufgebracht wurden.

Geeignete basische Träger für Hydrierkatalysatoren sind beispielsweise ß-Aluminiumoxid oder Magnesiumoxid/Aluminiumoxid-Gemische, wobei der Anteil des Magnesiumoxids vorzugsweise 5 bis 40 Gew.-% beträgt. Dabei kann der Magnesiumoxid und Aluminiumoxid enthaltende Träger amorph sein oder als Spinell vorliegen. Katalysatoren auf basischen Trägern erhält man technisch in an sich bekannter Weise. So gewinnt man z.B. Ruthenium auf basischen Träger durch Auftragen von wässrigen Rutheniumsalz-Lösungen, wie Rutheniumchlorid und Rutheniumnitrat auf den entsprechenden basischen Träger.

Die Konzentration der Metalle, insbesondere Ruthenium, auf den basischen Trägern beträgt in der Regel 0,1 bis 10 Gew.-%, bevorzugt 0,5 bis 5 Gew.-% und besonders bevorzugt 1 bis 4 Gew.-%.

Unter basischen Katalysatoren versteht man auch solche Hydrierkatalysatoren, die mit den oben genannten basischen Komponenten, wie Oxiden oder Hydroxiden von Alkali-, Erdalkali- und Seltenerdmetallen, dotiert werden. Bevorzugt enthalten basische Katalysatoren mindestens eine basische Komponente, wie Li₂O, Na₂O, K₂O, MgO, CaO, SrO oder BaO.

Der Anteil an basischen Komponenten, d.h. basischen Dotierungen in basischen Hydrierkatalysatoren beträgt in der Regel mehr als 0,5 Gew.-% und besonders bevorzugt mehr als 0,7 Gew.-% und besonders bevorzugt mehr als 1 Gew.-% bezogen auf die Gesamtmasse des basischen Hydrierkatalysators.

Die eingangs beschriebenen Hydrierkatalysatoren, die nicht wie oben beschrieben auf basischen Trägern aufgebracht wurden und/oder die 0,5 Gew.-% oder weniger basische Komponenten, d.h. basischen Dotierungen, bezogen auf die Gesamtmasse des Katalysators enthalten, werden im folgenden als nicht-basische Hydrierkatalysatoren bezeichnet.

Die reduktive Aminierung erfolgt üblicherweise bei Temperaturen von 50 bis 160°C und einem Druck von 50 bis 300 bar.

Die reduktive Aminierung kann diskontinuierlich oder bevorzugt kontinuierlich durchführt werden.

Die diskontinuierliche reduktive Aminierung kann beispielsweise in einem Rührautoklaven, einer Blasensäule oder einem Umlaufreaktor, wie etwa einem Strahlschlaufenreaktor, durchgeführt werden.

Bei der diskontinuierlichen reduktiven Aminierung wird üblicherweise eine Suspension von Carvonnitrilimin und Katalysator im Reaktor vorgelegt. Um einen hohen Umsatz und hohe Selektivität zu gewährleisten, wird die Suspension von Carvonnitrilimin und Katalysator mit Wasserstoff und dem Aminierungsmittel üblicherweise gut gemischt werden, z.B. durch einen Turbinenrührer in einem Autoklaven. Das suspendierte Katalysatormaterial kann mit Hilfe gebräuchlicher Techniken eingebracht und wieder abgetrennt werden (Sedimentation, Zentrifugation, Kuchenfiltration, Querstromfiltration). Der Katalysator kann einmal oder mehrmals verwendet werden.

Die Katalysatorkonzentration beträgt vorteilhaft 0,1 bis 50 Gew.-%, bevorzugt 0,5 bis 40 Gew.-%, besonders bevorzugt 1 bis 30 Gew.-%, insbesondere 5 bis 20 Gew.-%, jeweils bezogen auf das Gesamtgewicht der Suspension bestehend aus Carvonnitrilimin und Katalysator.

Die mittlere Katalysatorpartikelgröße beträgt vorteilhaft im Bereich von 0,001 bis 1 mm, bevorzugt im Bereich von 0,005 bis 0,5 mm, insbesondere 0,01 bis 0,25 mm. Gegebenenfalls kann eine Verdünnung der Edukte mit einem geeigneten, inerten Lösungsmittel, in dem 3-Imino-5-isopropenyl-2-methyl-cyclohexancarbonitril eine gute Löslichkeit aufweist, wie Tetrahydrofuran, Dioxan, N-Methylpyrrolidon, erfolgen.

Die kontinuierliche reduktive Aminierung kann beispielsweise in einem kontinuierlich betriebenen Rührautoklaven, einer kontinuierlich betriebenen Blasensäule, einem kontinuierlich betriebenen Umlaufreaktor, wie etwa einen Strahlschlaufenreaktor oder einem Festbettreaktor durchgeführt werden.

Die kontinuierliche, reduktive Aminierung wird vorzugsweise in einem Rohrreaktor mit Katalysatorfestbett durchgeführt.

Insbesondere ist für diese Reaktion ein Rohrreaktor mit Katalysatorfestbett geeignet. Die Katalysatorbelastung bei kontinuierlicher Fahrweise liegt typischerweise bei 0,01 bis 10, vorzugsweise von 0,05 bis 7, besonders bevorzugt von 0,1 bis 5 kg Carvonnitrilimin pro kg Katalysator und Stunde.

Bevorzugt erfolgt die reduktive Aminierung kontinuierlich in einem Rohrreaktor mit Katalysatorfestbett.

Die reduktive Aminierung, d.h. die Umsetzung des Carvonnitrilimin-haltigen Eduktstroms mit Wasserstoff und Ammoniak an Hydrierkatalysatoren kann in einem oder in mehreren voneinander getrennten Reaktionsräumen erfolgen.

Wird die reduktive Aminierung in nur einem Reaktionsraum durchgeführt, so ist üblicherweise das Temperaturprofil zwischen Reaktoreingang und Reaktorausgang weitestgehend konstant und durch die bei der reduktiven Aminierung frei werdende Reaktionswärme bestimmt.

Es ist jedoch auch möglich, ein Temperaturprofil zwischen Reaktoreingang und Reaktorausgang einzustellen. Die Ausbildung eines solchen Temperaturprofils kann dadurch realisiert werden, dass einzelne Bereiche des Reaktors getrennt und individuell voneinander einstellbar temperiert werden können. In einem solchen Falle ist es vorteilhaft, wenn die Temperatur zwischen Reaktoreingang und Reaktorausgang erhöht wird. Vorzugsweise beträgt die Temperatur am Reaktoreingang im Bereich von 50 bis 100°C, während die Temperatur am Reaktorausgang zwischen 100 und 160°C liegt. Das zunehmende Temperaturprofil zwischen Reaktoreingang und Reaktorausgang kann eine stetige Funktion sein oder in diskreten Schritten zunehmen.

In einer bevorzugten Ausführungsform erfolgt die reduktive Aminierung jedoch in zwei oder mehreren Stufen, wobei die Stufen in getrennten Reaktionsräumen erfolgen.

In einer besonders bevorzugten Ausführungsform wird die reduktive Aminierung in zwei Stufen durchgeführt, wobei die Stufen in getrennten Reaktionsräumen erfolgen.

Die erste Stufe (Stufe I) wird in der Regel in einem Temperaturbereich 50 bis 100°C, bevorzugt bei 55 bis 95°C und besonders bevorzugt bei 60 bis 90°C und bei einem Druck von 15 bis 300, vorzugsweise 20 bis 250 und besonders bevorzugt bei 30 bis 230 bar durchgeführt.

Die zweite Stufe (Stufe II) wird üblicherweise in einem Temperaturbereich von 70 bis 160°C, bevorzugt 75 bis 150°C und besonders bevorzugt bei 80 bis 140°C und bei einem Druck von 50 bis 300, vorzugsweise 80 bis 250 und besonders bevorzugt bei 100 bis 230 bar durchgeführt.

Beide Stufen werden üblicherweise jeweils in Druckbehältern, insbesondere in Festbettreaktoren durchgeführt.

Als Katalysatoren können in beide Stufen die eingangs beschriebenen nicht-basischen und/oder basischen Hydrierkatalysatoren eingesetzt werden, wobei bevorzugt ein nicht-basischer Katalysator eingesetzt wird, der Kobalt enthält.

In weiteren Ausführungsformen der Erfindung ist es möglich, sowohl die Stufe I als auch die Stufe II in weitere Teilstufen zu unterteilen, wobei auch die Teilstufen in jeweils getrennten Reaktionsräumen ausgeführt werden.

So ist es möglich, die Teilstufen der Stufe I in zwei oder mehreren Druckbehältern, insbesondere Festbettreaktoren, auszuführen.

Wie oben beschrieben werden die Teilstufen der Stufe I üblicherweise in einem Temperaturbereich von 50 bis 100°C und bei einem Druck von 15 bis 300 bar durchgeführt. Druck und Temperatur können in den Teilstufen gleich oder verschieden voneinander sein. Vorteilhafter Weise werden die Teilstufen bei gleicher Temperatur und gleichem Druck betrieben. Wenn die Teilstufen bei unterschiedlichen Temperaturen und Drücken betrieben werden ist es vorteilhaft, wenn Druck und Temperatur von Teilstufe zu Teilstufe zunehmen, d.h. dass der Druck und die Temperatur in der ersten Teilstufe am niedrigsten sein sollten.

In jeder Teilstufe können die eingangs beschriebenen nicht-basischen und/oder basischen Hydrierkatalysatoren eingesetzt werden, wobei bevorzugt nicht-basische Hydrierkatalysatoren eingesetzt werden.

In einer bevorzugten Ausführungsform werden in der ersten Teilstufe oder in den ersten Teilstufen der ersten Reaktionsstufe als nicht-basische Hydrierkatalysatoren selektive Hydrierkatalysatoren eingesetzt.

Aus Gründen der Verfahrensökonomie ist es vorteilhaft, wenn die Stufe I der reduktiven Aminierung aus nicht mehr als drei, vorzugsweise zwei und besonders bevorzugt einer Teilstufe besteht, da das Investment mit zunehmender Anzahl von Reaktoren zunimmt.

Wird die Stufe I der reduktiven Aminierung in nur einer Teilstufe durchgeführt, so ist es vorteilhaft, wenn die Basizität des Reaktionsgemisches erhöht wird, in dem die basische Verbindung mit dem Reaktionsgemisch nach dem Ausgang der Stufe I in Kontakt gebracht wird

Ferner ist es möglich die Stufe II der reduktiven Aminierung in weitere Teilstufen zu unterteilen, wobei die Teilstufen vorzugsweise in jeweils getrennten Reaktionsräumen ausgeführt werden.

Die Teilstufen der Stufe II der reduktiven Aminierung werden wie oben beschrieben üblicherweise in einem Temperaturbereich von 70 bis 160°C und bei einem Druck von 50 bis 300 bar durchgeführt. Vorzugsweise werden die Teilstufen der Stufe II der reduktiven Aminierung in zwei oder mehreren Druckbehältern, insbesondere Festbettreaktoren, ausgeführt.

Aus dem aus der reduktiven Aminierung erhaltenen Reaktionsaustrag werden NH₃ und Wasserstoff, gegebenenfalls unter Druck abgetrennt. Das so erhaltene Carvondiamin lässt sich beispielsweise durch eine fraktionierende Rektifikation isolieren.

Der aus der reduktiven Aminierung erhaltene Reaktionsaustrag kann aber auch durch Kristallisation oder chromatographisch aufgereinigt werden.

Durch das erfindungsgemäße Verfahren wird ein Gemisch von Stereoisomeren von 5-Isopropyl-3-aminomethyl-2-methyl-1-aminocyclohexan (Carvondiamin) erhalten. Das entstehende Isomerengemisch kann racemisch oder enantiomerenangereichert oder enantiomerenrein sein. Die Stereoisomerie wird z.T. in der Stufe der Hydrocyanierung festgelegt, vollständig jedoch in der reduktiven Aminierung/Hydrierung definiert.

Es ist möglich das Stereoisomerenverhältnis im Reaktionsaustrag zu regulieren, indem der Carvonnitrilimin-haltige Eduktstrom vor Einleitung in die Stufe I der reduktiven Aminierung geteilt wird. Ein Teil wird zusammen mit Wasserstoff und NH₃ in die Stufe I oder in die erste Teilstufe der Stufe I geleitet, während der andere Teil in eine spätere Stufe (Stufe II) oder Teilstufe der Stufe I oder Stufe II zugeführt wird. Bevorzugt wird ein Teil des Carvonnitrilimin-haltigen Eduktstroms in die zweite Stufe der reduktiven Aminierung (Stufe II) zugeführt bzw. in eine Teilstufe der zweiten Stufe der reduktiven Aminierung.

In der Regel wird durch die Teilung des Eduktstromes das thermodynamisch bevorzugte Produkt gebildet, so dass durch die Regelung der Teilung des Eduktstromes das Isomerenverhältnis eingestellt werden kann. In der zweiten Hydrierstufe kann nämlich eine teilweise Isomerisierung der chiralen Zentren erfolgen, indem aus der Aminogruppe wieder die prochirale Iminogruppe bzw. die Ketogruppe gebildet wird. Über Keto-Enoltautomerie bzw. Imino-Enamin-tautomerie kann dann auch die benachbarte Methylgruppe ihre räumliche Anordnung bezüglich der Ringebene ändern.

Eine weitere Steuerungsmöglichkeit des Stereoisomerenverhältnisses besteht in der Regulierung der Temperatur in der ersten Teilstufe der Stufe I. In beiden Fällen wird letztlich der Umsatz des Eduktstroms in der ersten Teilstufe der Stufe I geregelt. Je höher der Umsatz in der Stufe I bzw. der ersten Teilstufe der Stufe I ist, desto höher ist üblicherweise das der Anteil des kinetisch bevorzugten Produktes im Produktstrom.

In einer besonders bevorzugten Ausführungsform erhöht man die Basizität des Reaktionsgemisches während der Umsetzung von Carvonnitrilimin mit Ammoniak und Wasserstoff, in dem man das Reaktionsgemisch mit einer basischen Verbindung ungleich Ammoniak und/oder einem basischen Katalysator in Kontakt bringt, nachdem ein Teil des Carvonnitrilimins umgesetzt wurde.

Die Basizität des Reaktionsgemisches enthaltend Carvonnitrilimin, Ammoniak, Wasserstoff und den Hydrierkatalysator kann dadurch erhöht werden, dass man das Reaktionsgemisch mit einer basischen Verbindung in Kontakt bringt.

So kann die Basizität des Reaktionsgemisches dadurch erhöht werden, dass man dem Reaktionsgemisch eine basische Verbindung zufügt.

In einer weiteren Ausführungsform kann die Basizität des Reaktionsgemischs dadurch erhöht werden, dass man einen basischen Hydrierkatalysator mit dem Reaktionsgemisch in Kontakt bringt.

In dieser besonders bevorzugten Ausführungsform wird die Basizität des Reaktionsgemisches während der Umsetzung erhöht, in dem man das Reaktionsgemisch mit einer basischen Verbindung in Kontakt bringt, nachdem ein Teil des Carvonnitrilimin umgesetzt wurde.

Dabei erfolgt in der Regel die Erhöhung der Basizität durch Inkontaktbringen des Reaktionsgemischs mit der basischen Verbindung, nachdem 1 bis 95%, bevorzugt 5 bis 80% und besonders bevorzugt 10 bis 40% des Carvonnitrilimin im Reaktionsgemisch umgesetzt wurden.

Vor Erhöhung der Basizität werden dem Reaktionsgemisch in der Regel keine basischen Verbindungen zugegeben. Es ist jedoch möglich, dass das Reaktionsgemisch geringe Mengen basischer Verbindungen enthält. Vorzugsweise beträgt das Verhältnis der Masse der basischen Verbindung zur Masse des Carvonnitrilimin im Reaktionsgemisch vor der Erhöhung der Basizität jedoch weniger als 100 zu 1 000 000, vorzugsweise weniger als 50 zu 1 000 000.

Vor der Erhöhung der Basizität wird das Reaktionsgemisch üblicherweise mit nicht-basischen Katalysatoren in Kontakt gebracht.

Wird die reduktive Aminierung in nur einem Reaktionsraum durchgeführt, beispielsweise in einem Festbettreaktor, so kann die Erhöhung der Basizität durch das Inkontaktbringen des Reaktionsgemischs mit der basischen Verbindung in der Weise erfolgen, dass die Dosierung der basischen Verbindung zwischen dem Reaktoreingang, in den der Carvonnitrilimin-haltige Eduktstrom zusammen mit Ammoniak und Wasserstoff zugeführt wird, und dem Reaktorausgang erfolgt. Das Inkontaktbringen des Eduktstroms mit der basischen Verbindung erfolgt im Rahmen dieser besonders bevorzugten Ausführungsform nicht vor der reduktiven Aminierung.

Da wie oben beschrieben, die Reaktion bevorzugt unter einem hohen Druck erfolgt, ist es deshalb in der Regel erforderlich eine Dosierung der basischen Verbindung bei einem hohen Betriebsdruck in dem Reaktor vorzunehmen. Geeignete technische Vorrichtungen zur Dosierung von Stoffen unter Hochdruckbedingungen sind dem Fachmann bekannt. Insbesondere können Pumpen, wie Hochdruckpumpen bzw. Kolbenpumpen zur Dosierung von Stoffen unter Hochdruckbedingungen verwendet werden.

Es ist jedoch auch möglich, dass die Erhöhung der Basizität des Reaktionsgemisches durch das Inkontaktbringen mit einem basischen Katalysator in der Art erfolgt, dass zunächst der Carvonnitrilimin-haltige Eduktstrom mit Wasserstoff und Ammoniak über einen der eingangs beschriebenen nicht-basischen Hydrierkatalysatoren und nachfolgend über einen basischen Hydrierkatalysator geleitet wird. Dies kann dadurch realisiert werden, dass die Katalysatoren in geeigneter Weise geschichtet sind.

Vorteilhafter Weise wird am Übergang zwischen der Schicht des nicht-basischen Hydrierkatalysators und des basischen Hydrierkatalysators, wie oben beschrieben, eine basische Verbindung zudosiert, da die basischen Komponenten des Hydrierkatalysators mit zunehmender Betriebsdauer ausgewaschen werden können.

Wird die reduktive Aminierung jedoch in zwei Stufen durchgeführt, wobei die Stufen üblicherweise in getrennten Reaktionsräumen erfolgen, so kann die Erhöhung der Basizität des Reaktionsgemisches durch Inkontaktbringen des Reaktionsgemischs mit der basischen Verbindung bevorzugt erfolgen, indem zwischen dem Ausgang der Stufe I und dem Eingang der Stufe II eine Lösung einer basischen Verbindung zudosiert wird. Die Erhöhung der Basizität des Reaktionsgemisches durch das Inkontaktbringen mit einer basischen Verbindung kann aber auch in der Art erfolgen, dass in der Stufe I einer der eingangs beschriebenen nicht-basischen Hydrierkatalysatoren und in die Stufe II ein basischer Hydrierkatalysator eingesetzt wird.

Da die basischen Komponenten aus dem basischen Katalysator mit zunehmender Betriebsdauer ausgewaschen werden können, ist es vorteilhaft, wenn zwischen dem Ausgang der Stufe I und dem Eingang der Stufe II zusätzliche eine Lösung einer basischen Verbindung zudosiert wird.

In einer besonders bevorzugten Ausführungsform werden in die Stufe I als nicht-basische Hydrierkatalysatoren die eingangs beschriebenen selektiven Hydrierkatalysatoren eingesetzt.

Wird die Stufe I der reduktiven Aminierung in zwei oder mehr Teilstufen durchgeführt, so ist es empfehlenswert die Basizität des Reaktionsgemisches zu erhöhen, indem das Inkontaktbringen des Reaktionsgemischs mit der basischen Verbindung nach der ersten Teilstufe der Stufe I vorgenommen wird.

Vorzugsweise wird das Reaktionsgemisch mit der basischen Verbindung in Kontakt gebracht, in dem die basische Verbindung zwischen dem Ausgang einer Teilstufe und dem Eingang der darauffolgenden Teilstufe der Stufe I zudosiert wird. Vorteilhafterweise erfolgt die Dosierung der basischen Verbindung zwischen der ersten Teilstufe und der zweiten Teilstufe der Stufe I. Es ist aber auch möglich die basische Verbindung zwischen dem Ausgang und dem Eingang von zwei beliebig aufeinanderfolgenden Teilstufen zu dosieren. Die Dosierung der basischen Verbindung erfolgt in der Regel nicht vor der ersten Teilstufe der Stufe I.

Die Erhöhung der Basizität des Reaktionsgemischs durch das Inkontaktbringen mit einem basischen Hydrierkatalysator kann auch in der Art erfolgen, dass in der ersten oder in den ersten Teilstufen einer der eingangs beschriebenen nicht-basischen Hydrierkatalysatoren eingesetzt wird und in einer der folgenden Teilstufen ein basischer Hydrierkatalysator verwendet wird. Es ist auch denkbar, dass in den Teilstufen eine Schichtung von nicht-basischen Hydrierkatalysatoren und basischen Hydrierkatalysatoren erfolgt.

Weiterhin ist es vorteilhaft in die Teilstufen mit basischen Hydrierkatalysatoren zusätzlich die Dosierung einer Lösung einer basischen Verbindung vorzunehmen, um die Möglichkeit des Auswaschens der basischen Komponenten des basischen Hydrierkatalysators zu kompensieren.

Vorzugsweise sollte die Erhöhung der Basizität des Reaktionsgemischs durch das Inkontaktbringen des Reaktionsgemischs mit einer basischen Verbindung und/oder einem basischen Hydrierkatalysator vor der Stufe II erfolgen. Es ist jedoch auch möglich, dass das Inkontaktbringen des Reaktionsgemischs in einer der Teilstufen der zweiten Reaktionsstufe vorzunehmen. Dies kann in analoger Weise dadurch erfolgen, dass eine Lösung einer basischen Verbindung zwischen den Teilstufen der Stufe II zudosiert wird, oder hinter der ersten Teilstufe der Stufe II ein basischer Hydrierkatalysator eingesetzt wird.

Weiterhin ist eine Schichtung von Hydrierkatalysatoren und basischen Hydrierkatalysatoren in den Teilstufen der Stufe II möglich.

Die vorliegende Erfindung betrifft zudem die Verwendung von 5-Isopropyl-3-aminomethyl-2-methyl-1-amino-cyclohexan (Carvondiamin) als Härter für Epoxyharze, als Zwischenprodukt bei der Herstellung von Diisocyanaten, als Starter bei der Herstellung von Polyetherolen und/oder als Monomer für die Polyamidherstellung.

5-Isopropyl-3-aminomethyl-2-methyl-1-amino-cyclohexan (Carvondiamin) stellt einen alternativen Härter für Epoxyharze dar, der neue Möglichkeiten bei der Formulierung und Verarbeitung von Epoxyharzen ermöglicht und zur Regulierung des Eigenschaftsspektrums von Epoxyharzen eingesetzt werden kann. Carvondiamin weist, ähnlich wie IPDA, sowohl eine Amingruppe auf, die direkt am Ring gebunden ist, sowie eine Aminogruppe, die über eine Methylengruppe mit dem aliphatischen Ring verknüpft ist. Somit zeigt Carvondiamin ein ähnliches Reaktivitätsverhalten wie IPDA und kann in vielen Fällen als Substitut für IPDA eingesetzt werden.

5-Isopropyl-3-aminomethyl-2-methyl-1-amino-cyclohexan (Carvondiamin) kann auch als Zwischenprodukt bei der Herstellung von Carvondiisocyanat (5-Isopropyl-3-isocyanatomethyl-2-methyl-1-isocyanato-cyclohexan) der Formel (V) verwendet werden.

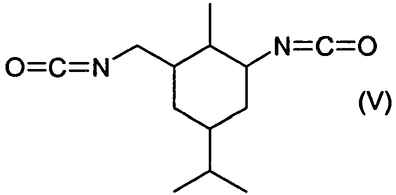

Dieses Diisocyanat ist für die Herstellung von lichtbeständigen Polyurethanen, beispielsweise als Lack oder Beschichtung, geeignet und bietet aufgrund seiner Struktur neue Formulierungsmöglichkeiten und damit Zugang zu neuen, interessanten Eigenschaftsprofilen. Carvondiisocyanat ist beispielsweise durch Umsetzung von Carvondiamin mit Phosgen erhältlich.

5-Isopropyl-3-aminomethyl-2-methyl-1-amino-cyclohexan (Carvondiamin) kann auch als Starter bei der Herstellung von Polyetherolen verwendet werden. 5-Isopropyl-3-aminomethyl-2-methyl-1-amino-cyclohexan (Carvondiamin) ist eine CH-acide Verbindung, die mit einer Base deprotoniert werden kann und an die nachfolgenden Alkylenoxide, wie Ethylenoxid, Propylenoxid und/oder Butylenoxid addiert werden können. Akoxilierte Diamine können beispielsweise als Katalysatoren in der PUR-Herstellung eingesetzt werden.

Weiterhin kann 5-Isopropyl-3-aminomethyl-2-methyl-1-amino-cyclohexan (Carvondiamin) als Monomer bei der Herstellung von Polyamiden eingesetzt werden. So kann 5-Isopropyl-3-aminomethyl-2-methyl-1-amino-cyclohexan (Carvondiamin) beispielsweise mit Dicarbonsäuren, wie beispielsweise Bernsteinsäure, Adipinsäure, Terephthalsäure und/oder Phthalsäure zu Polymeren umgesetzt werden.

Mittels der beschriebenen Erfindung ist es möglich eine hohe Carvondiamin-Ausbeute zu erzielen. Das Stereoisomerenverhältnis kann durch Einstellung der Reaktionsbedingungen reguliert werden.

Das dargestellte Verfahren kann mit einer hohen Raum-Zeit-Ausbeute betrieben werden. Die Bildung von störenden Nebenprodukten, insbesondere Dimeren, wird weitestgehend vermieden. Ein besonderes Merkmal des Verfahrens ist die Erreichung eines möglichst hohen Nitrilumsatzes bzw. Sättigungsgrades des Reaktionsproduktes, da für optimale Eigenschaften in Polymeren keine Nitrilamine, Aminoimine und Olefinreste zugegen sein dürfen. Teilgesättigte Verbindungen sind gemeinhin nur schwer von gesättigten abzutrennen.

Carvondiamin kann bei der Herstellung von Polymermaterialien, wie Epoxyharzen, Polyurethanen, Polyestern etc., genutzt werden, um das Eigenschaftsprofil dieser Polymermaterialien, beispielsweise in Hinblick auf Wetterbeständigkeit, Hydrolysebeständigkeit, Chemikalienbeständigkeit, Lichtbeständigkeit, elektrische und mechanische Eigenschaften zu regulieren und erlaubt somit höhere Variationsmöglichkeiten bei der Formulierung dieser Materialien.

Mittels der vorliegenden Erfindung konnte ein Diamin erhalten werden, das ähnlich wie IPDA, sowohl eine Amingruppe aufweist, die direkt am Ring gebunden ist, und eine Aminogruppe aufweist, die über eine Methylengruppe mit dem aliphatischen Ring verknüpft ist. Carvondiamin sollte deshalb ein ähnliches Reaktivitätsprofil wie IPDA aufweisen und das als Substitut für IPDA in vielen Anwendungen eingesetzt werden können.

Carvondiamin kann zudem auf Basis von nachwachsenden Rohstoffen hergestellt werden. Somit kann die Verwendung von Carvondiamin anstelle von Einsatzstoffen, die auf petrochemischer Basis hergestellt werden, die natürlichen Gas- und Ölreserven schonen. Der nachwachsende Rohstoff Carvon wird üblicherweise nicht als Nahrungsmittel bzw. als Rohstoff für die Lebensmittelindustrie eingesetzt, so dass dessen Einsatz nicht zu einer Verknappung von Lebensmitteln beitragen sollte.

Die Erfindung wird in den nachfolgenden Beispielen erläutert.

### Beispiele:

### Beispiel 1: Umsetzung von Carvon zu Carvonnitril

1379,4 g (9 mol) R-(-)- Carvon (5-Isoprenyl-2methyl-cyclohex-2-enon) mit einer Reinheit von 98% (5-Isoprenyl-2methyl-cyclohex-2-enon) wurden in einem 4000 ml Glaskolben und auf ca. 150°C unter rühren erwärmt. Bei 144°C wurden 18,4 g Na-Methylat 30% in Wasser (0,1 mol) zugegeben und anschließend unter Rühren eine Mischung von 1287,9 g (8,4 mol) R-(-)- Carvon 98% (5-Isoprenyl-2methyl-cyclohex-2-enon) und 330,5 g (12,15 mol) Blausäure (HCN) innerhalb von 8h bei 136-148°C zugetropft. Die Nachreaktionszeit betrug ca. eine Stunde. Anschließend wurde der HCN-Umsatz überprüft, der 99,5% betrug. Der gesamte Rohaustrag wog 3014 g und wies eine dunkelrote Farbe auf. Der Rohaustrag wurde über eine Vigreux-Kolonne destilliert. Als erste Fraktion wurde nicht umgesetztes Carvon zurückgewonnen (ca. 900 g), nach einer Mischfraktion (147,5 g) wurden 1809,8 g Carvonnitril mit einer Reinheit von 98-99% (Gaschromatographie) erhalten.

Die Ausbeute unter Einrechnung des in der Mischfraktion enthaltenen Wertproduktes betrug 93,7% bezogen auf HCN.

### Beispiel 2: Umsetzung von Carvonnitril zu Carvondiamin

Die verwendete Apparatur bestand aus 8 seriell verschalteten Rohrreaktoren (2 Rohre mit einer Abmessung von 1500x6x1 mm (C1-C2) und 6 Rohre mit einer Abmessung von jeweils 2000x8x1,5 mm (C3-C8)). Die ersten beiden Reaktoren C1-C2 wurden mit 15,7 g TiO₂ Strängen mit einem Strang-Durchmesser von 1,5mm befüllt, die übrigen 6 Reaktoren wurden mit je ca. 85 g eines Hydrierkatalysators (Mn₃O₄ 5-6,2% Na₂O 0-0,5%, H₃PO₄ 2,8-3,8%, Rest Co + CoO) befüllt, der mit Wasserstoff bei 280°C bei einem Druck von 1 bar für 24 Stunden reduziert wurde.

Die Temperatur wurde über Ölmantelheizung in den Reaktoren C1 und C2 auf 60°C, in den Reaktoren C3-C4 auf 90°C, in den Reaktoren C5-C6 auf 115°C und in den Reaktoren C7-C8 auf 130°C eingestellt. Zwischen den Reaktoren C2 und C3 wurde Wasserstoff unter Druck (230 bar) in die Reaktionsmischung eingespeist.

Stündlich pumpte man 23 g eines Gemisches von THF und Carvonnitril im Verhältnis 1:1 zusammen mit 73 g NH₃ in den ersten Reaktor (C1) und führte vor dem Reaktor C3 noch 17 Normliter/h Wasserstoff zu. Der Reaktionsaustrag wurde über ein Regelventil entspannt. In einem nachgeschalteten Phasenscheider wurde anschließend Wasserstoff abgetrennt und Ammoniak abgedampft.

Eine Probe des Reaktionsaustrags wurde nach einer Betriebsdauer von 21,5 Stunden mittels Gaschromatographie analysiert. Der Reaktionsaustrag wies folgende Zusammensetzung auf (Die Gehalte der Verbindungen mittels Gaschromatographie wurden als Flächenprozente ermittelt. Hierbei beziehen sich die Flächenprozente der Signale auf die Gesamtfläche unterhalb der gemessenen Signale mit Ausnahme des Wassersignals und des Ammoniaksignals.):
3,86% eines bicyclischen Amins der allgemeinen Formel VI,
0,68% eines isomeren bicyclischen Amins der Formel VI, 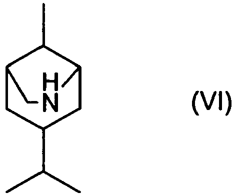
und in Summe 89.16% von Carvondiamin als Mischung von 4 Diastereoisomeren.

Des Weiteren waren 1,5% unvollständig hydrierte Verbindungen enthalten, die entweder ungesättigte CN oder CC Bindungen besaßen.

Das innerhalb einer Betriebsdauer von 45 Stunden angefallene und gesammelte Rohprodukt wurde von THF befreit und durch Destillation gereinigt.

Bei einem Betriebsdruck von 10 mbar destillierte das Wertprodukt Carvondiamin bei 129-130°C über.

Aus 1024 g THF-armen Rohprodukt wurden 791 g Carvondiamin als Isomerengemisch mit einem Gehalt an Carvondiamin von über 99% (GC-Analyse) erhalten, was einer Ausbeute von 74% bezogen auf 1035 g eingesetztes Carvonnitril entspricht.

Das Isomerengemisch von Carvondiamin wurde durch GC-MS, NMR und Elementaranalyse charakterisiert.

Im GC-MS nach Methode 30m db1701 1 Mikrometer 80°C Anfangstemperatur, Temperaturrampe 10°C/min auf 280°C wurden 4 Hauptpeaks, die den entsprechenden reinen Diastereoisomeren von Carvondiamin entsprechen, detektiert, Retentionszeit 17.41 min. (12,4 FI-%), 17,45 min. (11,4 FI.-%), 17,72 min. (17,1 FI-%) und 18,23 min. (58,5 FI.-%). Die Reinheit betrug also 99,4% nach GC.

¹H-NMR (500 MHz, DMSO): 0,6-0,8 (Teil eines dedublierten Dubletts, 1,5 H), 0,8-0,95 (Signalhaufen, 8,5 H), 1,0-1,5 (Signalhaufen, 8 H), 1,55-1,92 (Signalhaufen, 3 H), 2,25-2,85 (m, 3 H).

Es wurden folgende Fragmentverteilungen erhalten (M+ = 184 entspricht jeweils dem Molpeak):
Peak Nr. 1:
   m/z (%) = 18 (5), 27 (8), 28 (10), 29 (10), 30 (93), 39 (10), 41 (33), 42 (14), 43 (41), 44 (16), 45 (3), 53 (9), 54 (6), 55 (46), 56 (81), 57 (54), 58 (7), 65 (3), 67 (15), 68 (12), 69 (27), 70 (30), 71 (19), 72 (5), 77 (6), 79 (14), 80 (6), 81 (29), 82 (41), 83 (13), 84 (35), 85 (4), 91 (6), 93 (8), 94 (9), 95 (46), 96 (16), 97(6), 98 (28), 99 (3), 106 (3), 107 (17), 108 (5), 109 (8), 110 (8), 111 (4), 112 (15), 121 (3), 122 (3), 123 (4), 124 (100), 125 (15), 126 (3), 135 (4), 136 (6), 137 (4), 138 (19), 139 (3), 141 (29), 142 (4), 150 (10), 152 (8), 154 (19), 155 (14), 184 (5)
Peak Nr. 2:
   m/z (%) = 18 (4), 27 (6), 28(5), 29 (8), 30 (70), 39 (7), 41 (25), 42 (10), 43 (31), 44 (12), 45 (4), 53 (7), 54 (5), 55 (38), 56 (38), 57 (31), 58 (6), 65 (3), 67 (10), 68 (8), 69 (21), 70 (24), 71 (16), 72 (5), 77 (5), 79 (11), 80 (3), 81 (21), 82 (28), 83 (10), 84 (35), 85 (5), 91 (5), 93 (6), 94 (6), 95 (29), 96 (10), 97 (5), 96 (30), 99 (3), 101 (3), 107 (11), 108 (4), 109 (6), 110 (8), 111 (4), 112 (14), 123 (3), 124 (100), 125 (21), 126 (4), 137 (3), 138 (14), 141 (30), 142 (6), 150 (3), 152 (6), 154 (26), 155 (9), 184 (3)
Peak Nr. 3:
   m/z (%) = 18 (3), 27 (5), 28 (8), 29 (6), 30 (54), 39 (6), 41 822), 42 (10), 43 (28), 44 (9), 53 (6), 54 (4), 55 (25), 56 (36), 57 (16), 58 (4), 67 (10), 68 (8), 69 (16), 70 (25), 71 (70), 72 (7), 77 (4), 79 (9), 80 84), 81 (16), 82 (32), 83 (9), 84 (21), 91 (4), 93 (5), 94 (5), 95 (28), 96 (13), 97 (3), 98 (15), 107 (9), 108 (3), 109 (5), 110 (12), 111 (3), 112 (8), 138 (8), 141 (11), 152 (6), 154 (11), 155 (6)
Peak Nr. 4:
   m/z (%) = 18 (4), 27 (7), 28 (11), 29 (8), 30 (68), 39 (8), 41 (29), 42 (12), 43 (36), 44 (13), 45 (3), 53 (8), 54 (5), 55 (33), 56 (100), 57 (51), 58 (6), 67 (13), 68 (11), 69 (21), 70 (32), 71 (27), 72 (5), 77 (5), 79 (12), 80 (5), 81 (23), 82 (42), 83 (12), 84 (28), 85 (3), 91 (6), 93 (7), 94 (8), 95 (37), 96 (16), 97 (5), 98 (20), 107(14), 108 (4), 109 (7), 111 (4), 112 (17), 121 (3), 123 (4), 124 (84), 125 (21), 126 (4), 135 (4), 136 (5), 137 (3), 138 (15), 141 (22), 142 (3), 150 (5), 152 (10), 154 (30), 155 (17), 169 (3), 184 (4)

Zur Bestimmung der Elementaranalysen wurde ein Analysenautomat Vario EI III der Firma Elementar verwendet.

Die Elementaranalyse ergab:
C = 71,3 (erwartet: 71,7); N = 15,6 (erwartet: 15,2); H = 13,4 (erwartet: 13,1) g/100g

### Beispiel 3: Vergleich der Härtung von DGEBA (Diglycidylether von Bisphenol A) mit Isophorondiamin (IPDA) bzw. Carvondiamin

Die verwendeten Formulierungen wiesen folgende Zusammensetzung auf:
Formulierung 1: 17,96 g DGEBA, 4,10 g IPDA
Formulierung 2: 29,51 g DGEBA, 7,27g Carvondiamin

Vor der Härtung wurden die Komponenten der Formulierung für zwei Minuten bei 3000 U/min (Speedmixer DAC 150 FVZ) durchmischt.

Nach der Durchmischung wurden ca. 15,5 g der jeweiligen Formulierung in Aluminiumschalen mit den Abmessungen 7x3.5x3 cm (LxBxH) gegossen, um Harzplatten mit einer Schichtdicke von 3 - 4 mm herzustellen. Die Harzplatten wurden für jeweils 30 min bei 60°C, 80°C, 100°C, 120°C, 140°C, 160°C und 180°C gehärtet.

Parallel zur Herstellung der Platten wurde die Aushärtung der jeweiligen Formulierung mittels DSC untersucht. Hierzu wurden üblicherweise 3 bis 10 mg Harzformulierung eingesetzt. Die Messungen erfolgten an der Luft auf einem DSC822^{e} Gerät der Firma Mettler Toledo im dynamischen Modus mit einer Heizrate von 10 °C/min. Onset-Temperaturen Tₒ (Tangenten-Onset der Exothermie-Peaks), Peak-Maxima Tₘₐₓ und Reaktionsenthalpien ΔH der Polymerisationsreaktionen sind in Tabelle 1 zusammengefasst.

Zur Bestimmung der Glasübergangstemperaturen wurden den gehärteten Harzplatten Proben, üblicherweise einige mg, entnommen und hieraus mittels DSC (3 Heizsegmente mit einer Heizrate von 30 °C/min, Bestimmung aus dem Mittelwert der Segmente 2 und 3) die Glasübergangstemperaturen T_{g} bestimmt.

Des weiteren wurden durch eine thermogravimetrische Analyse (TGA) im Stickstoffstrom die Zersetzung des Polymers untersucht und der Zeitpunkt des 5 %igen Massenverlustes (5%-Abbau) bestimmt. Die Messungen wurden mit einem TGA/SDTA851^{e} Gerät der Firma Mettler Toledo durchgeführt.

Die Topfzeit bei 100°C wurde mit Hilfe eines GELNORM^{®}-RVN Topfzeitmessgeräts ermittelt, indem die relativen Viskositäten bei dieser Temperatur aufgenommen wurden. Die Daten liefern einen wichtigen Hinweis auf die Dauer der Härtung.

Die Produkte wurden mit DSC und TGA untersucht.

**Tabelle1: Härtung von DGEBA (9) mit Isophorondiamin (1) bzw. Carvondiamin**

| Formulierung | Menge | Menge | DSC Daten | | | | Platten | TGA | Topfzeit |
|---|---|---|---|---|---|---|---|---|---|
| | Harz [g] | Härter [g] | Tₒ [°C] | Tₘₐₓ [°C] | ΔH [J/g] | T_{g} [°C] | Aussehen | 5% Abbau [°C] | bei 100°C [sec] |
| 1 | 17,96 | 4,10 | 59 | 108 | 426 | 160 | Klar, transparent, fest | 355 | 490 - 620 |
| 2 | 29,51 | 7,27 | 82 | 118 | 390 | 159 | Klar, transparent, fest | 355 | 540 - 580 |

## Patentansprüche

1. 5-Isopropyl-3-aminomethyl-2-methyl-1-amino-cyclohexan (Carvondiamin) der Formel (I).

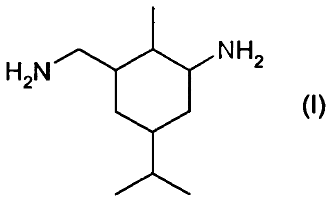

2. Verfahren zur Herstellung von Carvondiamin der Formel (I) durch
a) Umsetzung von Carvon der Formel (II) 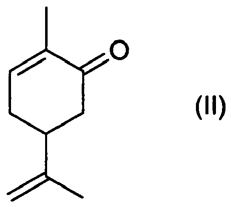 mit Cyanwasserstoff in Gegenwart eines basischen Katalysators zu Carvonnitril der Formel (III), 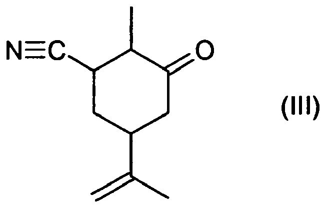
b) Umsetzung des in Stufe a) erhaltenen Carvonnitrils mit Ammoniak in Gegenwart eines Iminbildungskatalysators zu Carvonnitrilimin der Formel (IV), und 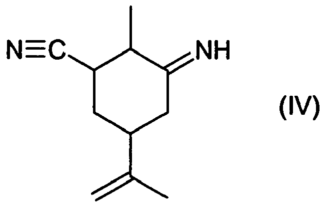
c) Umsetzung des in Stufe b) erhaltenen Carvonnitrilimin-haltigen Reaktionsgemisches mit Wasserstoff und Ammoniak an Hydrierkatalysatoren.

3. Verfahren nach Anspruch 2, **dadurch gekennzeichnet, dass** als basischer Katalysator in Stufe a) Natriumhydroxid, Natriumcyanid oder Natriummethylat verwendet wird.

4. Verfahren nach mindestens einem der Ansprüche 2 bis 3, **dadurch gekennzeichnet, dass** als Iminbildungskatalysator ein oder mehrere acide Metalloxidkatalysatoren, wie Aluminiumoxid, Titandioxid, Zirkoniumdioxid und Siliciumdioxid, verwendet werden.

5. Verfahren nach mindestens einem der Ansprüche 2 bis 4, **dadurch gekennzeichnet, dass** die Stufe c) in Gegenwart einer basischen Verbindung ungleich Ammoniak und/oder einem basischen Katalysator durchgeführt wird.

6. Verfahren nach mindestens einem der Ansprüche 2 bis 5, **dadurch gekennzeichnet, dass** man die Basizität des Reaktionsgemisches in Stufe c) während der Umsetzung erhöht, in dem man das Reaktionsgemisch mit einer basischen Verbindung ungleich Ammoniak und/oder einem basischen Katalysator in Kontakt bringt, nachdem ein Teil des Carvonnitrilimins umgesetzt wurde.

7. Verfahren nach Anspruch 6, **dadurch gekennzeichnet, dass** man vor Erhöhung der Basizität einen nicht-basischen Hydrierkatalysator einsetzt.

8. Verfahren nach mindestes einem der Ansprüche 6 bis 7, **dadurch gekennzeichnet, dass** man die Basizität des Reaktionsgemisches erhöht, in dem man eine basische Verbindung als Lösung zufügt und dass man die Menge der zugefügten basischen Verbindung als Lösung so wählt, dass das Verhältnis der Masse der zugefügten basischen Verbindung zur Masse des Carvonnitriliminim Eduktstrom 100 bis 10 000 zu 1 000 000 beträgt.

9. Verfahren nach mindestens einem der Ansprüche 6 bis 8, **dadurch gekennzeichnet, dass** man die Basizität des Reaktionsgemisches erhöht, in dem man als basische Verbindungen einen basischen Hydrierkatalysator einsetzt, wobei der Anteil von basischen Komponenten im basischen Hydrierkatalysator mindestens 0,5 Gew.-% bezogen auf die Gesamtmasse des basischen Hydrierkatalysators beträgt und/oder der Hydrierkatalysator auf einem basischen Träger geträgert wird.

10. Verfahren nach mindestens einem der Ansprüche 2 bis 9 **dadurch gekennzeichnet, dass** man einen kobalthaltigen Hydrierkatalysator verwendet.

11. Verfahren nach mindestens einem der Ansprüche 2 bis 10, **dadurch gekennzeichnet, dass** man die Umsetzung in Stufe c) in zwei Stufen (Stufe I und Stufe II) durchführt.

12. Verfahren nach Anspruch 11, **dadurch gekennzeichnet, dass** man die Stufe I in einem Temperaturbereich von 50 bis 100°C bei einem Druck von 15 bis 300 bar durchführt und die Stufe II in einem Temperaturbereich von 70 bis 160°C bei einem Druck von 50 bis 300 bar durchführt.

13. Verfahren nach Anspruch 12, **dadurch gekennzeichnet, dass** man in Stufe I einen Ruthenium- und/oder Rhodium-haltigen Katalysator verwendet.

14. Verfahren nach mindestens einem der Ansprüche 11 bis 13, **dadurch gekennzeichnet, dass** man das Reaktionsgemisch mit der basischen Verbindung nach Stufe I in Kontakt bringt.

15. Verfahren nach mindestens einem der Ansprüche 11 bis 13, **dadurch gekennzeichnet, dass** man die Stufe I und/oder die Stufe II in zwei oder mehreren Teilstufen durchführt, wobei man das Reaktionsgemisch mit der basischen Verbindung frühestens nach der ersten Teilstufe der Stufe I in Kontakt bringt.

16. Verfahren nach mindestens einem der Ansprüche 11 bis 13, **dadurch gekennzeichnet, dass** man den Eduktstrom aufteilt, indem man einen Teil des Eduktstroms in die Stufe I und einen Teil des Eduktstroms direkt in die Stufe II leitet.

17. Verfahren nach mindestens einem der Ansprüche 2 bis 16, **dadurch gekennzeichnet, dass** man die Basizität erhöht, nachdem 5 bis 80% Carvonnitrilimins umgesetzt wurden.

18. Verfahren nach mindestens einem der vorherigen Ansprüche, **dadurch gekennzeichnet, dass** man ein Carvon auf Basis von nachwachsenden Rohstoffen in das Verfahren einsetzt.

19. Verwendung von Carvondiamin der Formel (I) als Härter für Epoxyharze, als Zwischenprodukt bei der Herstellung von Diisocyanaten, als Starter bei der Herstellung von Polyetherolen und/oder als Monomer für die Polyamidherstellung.

## Claims

1. 5-Isopropyl-3-aminomethyl-2-methyl-1-aminocyclohexane (carvonediamine) of
the formula (I)

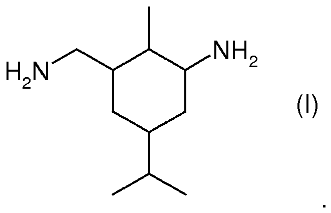

2. A process for preparing carvonediamine of the formula (I) by
a) reacting carvone of the formula (II) 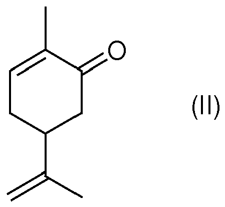 with hydrogen cyanide in the presence of a basic catalyst to give carvonenitrile of the formula (III) 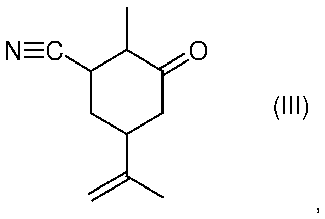
b) reacting the carvonenitrile obtained in stage a) with ammonia in the presence of an imine formation catalyst to give carvonenitrile imine of the formula (IV), and 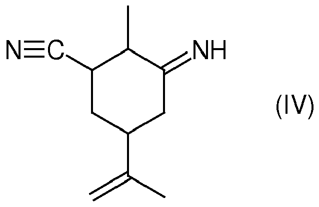
c) reacting the carvonenitrile imine-containing reaction mixture obtained in stage b) with hydrogen and ammonia over hydrogenation catalysts.

3. The process according to claim 2, wherein the basic catalyst used in stage a) is sodium hydroxide, sodium cyanide or sodium methoxide.

4. The process according to at least one of claims 2 and 3, wherein the imine formation catalyst used comprises one or more acidic metal oxide catalysts such as aluminum oxide, titanium dioxide, zirconium dioxide and silicon dioxide.

5. The process according to at least one of claims 2 to 4, wherein stage c) is performed in the presence of a basic compound other than ammonia and/or a basic catalyst.

6. The process according to at least one of claims 2 to 5, wherein the basicity of the reaction mixture is increased in stage c) during the reaction by contacting the reaction mixture with a basic compound other than ammonia and/or a basic catalyst once a portion of the carvonenitrile imine has been converted.

7. The process according to claim 6, wherein the increase in basicity is preceded by use of a nonbasic hydrogenation catalyst.

8. The process according to at least one of claims 6 and 7, wherein the basicity of the reaction mixture is increased by adding a basic compound as a solution and selecting the amount of the basic compound added as a solution such that the ratio of the mass of the basic compound added to the mass of the carvonenitrile imine in the reactant stream is 100 to 10 000:1 000 000.

9. The process according to at least one of claims 6 to 8, wherein the basicity of the reaction mixture is increased by using, as basic compounds, a basic hydrogenation catalyst, where the proportion of basic components in the basic hydrogenation catalyst is at least 0.5% by weight based on the total mass of the basic hydrogenation catalyst and/or the hydrogenation catalyst is supported on a basic support.

10. The process according to at least one of claims 2 to 9, wherein a cobalt-containing hydrogenation catalyst is used.

11. The process according to at least one of claims 2 to 10, wherein the reaction in stage c) is performed in two stages (stage I and stage II).

12. The process according to claim 11, wherein stage I is performed within a temperature range of 50 to 100°C at a pressure of 15 to 300 bar, and stage II within a temperature range of 70 to 160°C at a pressure of 50 to 300 bar.

13. The process according to claim 12, wherein a ruthenium- and/or rhodium-containing catalyst is used in stage I.

14. The process according to at least one of claims 11 to 13, wherein the reaction mixture is contacted with the basic compound after stage I.

15. The process according to at least one of claims 11 to 13, wherein stage I and/or stage II is performed in two or more component stages, the reaction mixture being contacted with the basic compound no earlier than after the first component stage of stage I.

16. The process according to at least one of claims 11 to 13, wherein the reactant stream is divided by passing a portion of the reactant stream into stage I and a portion of the reactant stream directly into stage II.

17. The process according to at least one of claims 2 to 16, wherein the basicity is increased once 5 to 80% of carvonenitrile imine has been converted.

18. The process according to at least one of the preceding claims, wherein a carvone based on renewable raw materials is used in the process.

19. The use of carvonediamine of the formula (I) as a hardener for epoxy resins, as an intermediate in the preparation of diisocyanates, as a starter in the preparation of polyetherols and/or as a monomer for polyamide preparation.

## Revendications

1. 5-isopropyl-3-aminométhyl-2-méthyl-1-aminocyclohexane (carvone-diamine) de formule (I).

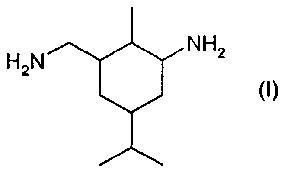

2. Procédé pour la préparation de carvone-diamine de formule (I) par
a) transformation de carvone de formule (II) 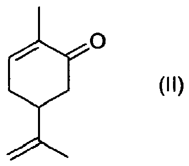 avec de l'acide cyanhydrique en présence d'un catalyseur basique en carvone-nitrile de formule (III), 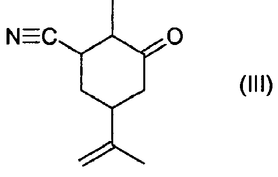
b) transformation du carvone-nitrile obtenu dans l'étape a) avec de l'ammoniaque en présence d'un catalyseur de formation d'imine en carvone-nitrile-imine de formule (IV), et 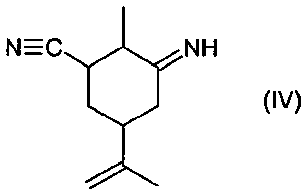
c) transformation du mélange réactionnel contenant la carvone-nitrile-imine obtenue dans l'étape b) avec de l'hydrogène et de l'ammoniac sur des catalyseurs d'hydrogénation.

3. Procédé selon la revendication 2, **caractérisé en ce qu'**on utilise, comme catalyseur basique dans l'étape a), de l'hydroxyde de sodium, du cyanure de sodium ou du méthylate de sodium.

4. Procédé selon au moins l'une quelconque des revendications 2 à 3, **caractérisé en ce qu'**on utilise comme catalyseur de formation d'imine un ou plusieurs catalyseurs acides de type oxyde de métal, tels que l'oxyde d'aluminium, le dioxyde de titane, le dioxyde de zirconium et le dioxyde de silicium.

5. Procédé selon au moins l'une quelconque des revendications 2 à 4, **caractérisé en ce que** l'étape c) est réalisée en présence d'un composé basique différent de l'ammoniaque et/ou d'un catalyseur basique.

6. Procédé selon au moins l'une quelconque des revendications 2 à 5, **caractérisé en ce qu'**on augmente la basicité du mélange réactionnel dans l'étape c) pendant la transformation, **en ce qu'**on met en contact le mélange réactionnel avec un composé basique différent de l'ammoniaque et/ou un catalyseur basique, après qu'une partie de la carvone-nitrile-imine a été transformée.

7. Procédé selon la revendication 6, **caractérisé en ce qu'**on utilise un catalyseur d'hydrogénation non basique avant l'augmentation de la basicité.

8. Procédé selon au moins l'une quelconque des revendications 6 à 7, **caractérisé en ce qu'**on augmente la basicité du mélange réactionnel **en ce qu'**on ajoute un composé basique sous forme de solution et **en ce qu'**on choisit la quantité de composé basique ajouté sous forme de solution de manière telle que le rapport de la masse de composé basique ajouté à la masse du flux de produits de départ de carvone-nitrile-imine est de 100:10 000 à 1 000 000.

9. Procédé selon au moins l'une quelconque des revendications 6 à 8, **caractérisé en ce qu'**on augmente la basicité du mélange réactionnel **en ce qu'**on utilise comme composés basiques un catalyseur d'hydrogénation basique, la proportion de composants basiques dans le catalyseur d'hydrogénation basique étant d'au moins 0,5% en poids par rapport à la masse totale du catalyseur d'hydrogénation basique et/ou le catalyseur d'hydrogénation étant supporté sur un support basique.

10. Procédé selon au moins l'une quelconque des revendications 2 à 9, **caractérisé en ce qu'**on utilise un catalyseur d'hydrogénation contenant du cobalt.

11. Procédé selon au moins l'une quelconque des revendications 2 à 10, **caractérisé en ce qu'**on réalise la transformation dans l'étape c) en deux étapes (étape I et étape II).

12. Procédé selon la revendication 11, **caractérisé en ce qu'**on réalise l'étape I dans une plage de température de 50 à 100°C à une pression de 15 à 300 bars et on réalise l'étape II dans une plage de température de 70 à 160°C à une pression de 50 à 300 bars.

13. Procédé selon la revendication 12, **caractérisé en ce qu'**on utilise, dans l'étape I, un catalyseur contenant du ruthénium et/ou du rhodium.

14. Procédé selon au moins l'une quelconque des revendications 11 à 13, **caractérisé en ce qu'**on met en contact le mélange réactionnel avec le composé basique après l'étape I.

15. Procédé selon au moins l'une quelconque des revendications 11 à 13, **caractérisé en ce qu'**on réalise l'étape I et/ou l'étape II en deux étapes partielles ou plus, le mélange réactionnel étant mis en contact avec le composé basique au plus tôt après la première étape partielle de l'étape I.

16. Procédé selon au moins l'une quelconque des revendications 11 à 13, **caractérisé en ce qu'**on répartit le flux de produits de départ **en ce qu'**on guide une partie du flux de produits de départ dans l'étape I et une partie du flux de produits de départ directement dans l'étape II.

17. Procédé selon au moins l'une quelconque des revendications 2 à 16, **caractérisé en ce qu'**on augmente la basicité après avoir transformé 5 à 80% de la carvone-nitrile-imine.

18. Procédé selon au moins l'une quelconque des revendications précédentes, **caractérisé en ce qu'**on utilise la carvone à base de matières premières renouvelables dans le procédé.

19. Utilisation de carvone-diamine de formule (I) comme durcisseur pour des résines époxy, en tant que produit intermédiaire lors de la préparation de diisocyanates, comme initiateur lors de la préparation de polyétherols et/ou comme monomère pour la préparation de polyamide.
